# EUROPEAN PATENT SPECIFICATION

(11) **EP 1 545 815 B1**
(45) Date of publication and mention of the grant of the patent: **01.03.2006**
(21) Application number: 03794963.3
(22) Date of filing: 20.08.2003
(51) Int. Cl.: B22F 1/02, B22F 9/26, B22F 3/11

(54) **NI-COATED TI POWDERS**
NI-BESCHICHTETE TI-PULVER
POUDRES DE TI ENDUITES DE NI

(30) Priority: 10.09.2002 EP 02078736; 12.09.2002 US 409990 P
(43) Date of publication of application: 29.06.2005
(73) Proprietor: Umicore, 1000 Brussels (BE)
(72) Inventor: YUAN, Ding, Edmonton, AB T6R 1A1 (CA); AMINIAN, Hossein, St. Albert, AB T8N 6S5 (CA)
(86) International application number: PCT/EP2003/009552
(87) International publication number: WO 2004/024373

(56) References cited:
- WO-A-01/87370
- US-A- 2 853 403
- PATENT ABSTRACTS OF JAPAN vol. 018, no. 632 (C-1280), 2 December 1994 (1994-12-02) -& JP 06 240381 A (KAWASAKI STEEL CORP), 30 August 1994 (1994-08-30)
- PATENT ABSTRACTS OF JAPAN vol. 1997, no. 02, 28 February 1997 (1997-02-28) -& JP 08 284622 A (MITSUBISHI MATERIALS CORP), 29 October 1996 (1996-10-29)
- DATABASE WPI Section Ch, Week 0155 Derwent Publications Ltd., London, GB; Class M,Page 22, AN 2001-501429 XP002228912 -& RU 2 170 645 C (DAMBAEV G TS ET AL), 20 July 2001 (2001-07-20)

## Description

The present invention relates to coated powder, comprising a Ti-bearing core and a Ni-bearing coating, which can be used for the production of porous Ni-Ti alloys.

Such a porous Ni-Ti alloy article is described in U.S. Pat. No. 5,986,169. The article has a porosity of 8 to 90 % and is defined by a network of interconnected passageways extending throughout. The network exhibits an isotropic permeability permitting complete migration of fluids. The material is elastically deformable. These characteristics render it useful in biomedical and other applications. For producing the porous article, the so-called self-propagating high-temperature synthesis (SHS) method is used in which the alloy is produced by means of a layered combustion which exploits exothermic heat emitted during interaction of elemental nickel and titanium powders.

U.S. Pat. No. 2,853,403 describes a method for producing composite metal powders. This method consists of dispersing, in solid form, particles of one or more metals of interest as nuclei in an ammoniated solution in which another metal of interest having different chemical and/or physical properties is present as a soluble salt, and precipitating the latter metal from the solution by gas reduction to form composite metal particles in which the dispersed metal particles are coated by the precipitated metal. This method was however never specifically applied for the manufacture of Ni-coated Ti powder.

In JP06-240381 Ti powder is coated with Ni by mechnical cladding, up to a weight level of 15% Ni, and the obtained powder is further kneaded, molded, degreased and sintered. In JP08-284622 composite powders for flame spray used for the cladding layer of an engine are made of Ti alloys coated with Ni, by stirring Ti powder with Ni powder finer than the Ti powder. The atomic ratio Ni:Ti is limited to 0.2.

In WO 01/87370 Ti, Ni and TiNi powders are mixed to produce a mixture to be sintered as a porous SHS body where the Ni:Ti ratio is 1. The use of coated powders is however not disclosed.

The use of elemental Ni and Ti powders renders the production process sensitive to segregation problems, resulting in composition fluctuations and/or non-homogeneous porosity. Composition fluctuations can in turn lead to the formation of unwanted secondary phases.

According to the invention, the above drawbacks can be overcome using coated powder, comprising a metallic Ti core and a metallic Ni coating, characterised by a Ni:Ti atomic ratio of more than 0.5, preferably between 0.9 and 1.1, and more preferably between 0.96 and 1.04. Atomic ratios of more than 0.5 to about 1 are preferred as this avoids the formation of secondary phases and yields an alloy with better mechanical properties.

It may be useful to mix Ti-bearing powder or Ni-bearing powder with the coated powder so as to obtain a Ni:Ti atomic ratio of the mixture of between 0.9 and 1.1, and preferably between 0.99 and 1.01. This procedure allows for easy adjustments to the Ni:Ti ratio.

The particle size of the powders should preferably be finer than 150 mesh.

Above described powders can be used for the manufacture of sintered objects, possibly using the SHS technique.

Another object of the invention concerns a process of manufacturing a coated powder comprising the steps of:
- providing for suitable quantities of a Ti powder and of a Ni salt bearing aqueous solution;
- feeding said powder and said solution in an autoclave together with a quantity of NH₄OH, and, optionally, with a quantity of ammonium salts;
- precipitating the Ni onto the Ti powder by hydrogen reduction;
- washing, filtering and drying the slurry obtained, thereby obtaining a Ni-coated Ti powder.

The Ni is preferably precipitated onto the Ti powder at a temperature of at least 100 °C and a hydrogen pressure in the autoclave of at least 1.4 MPa.

Powders obtained by this coating process are, as such or after mixing with Ni-bearing or Ti-bearing powders, suitable for SHS sintering of objects.

By using coated powders, local fluctuations in composition are limited and well under control. Ni-coated Ti powder also decreases the diffusion distance between the Ni and Ti atoms, which may eliminate or reduce the formation of unwanted intermetallic compounds such as Ni₃Ti and NiTi₂. It has been found that the porosity of the porous titanium-nickel produced by SHS starting from Ni-coated Ti powder is more homogeneous throughout the sample compared to starting with elemental powders. It has also been found that the compactibility of Ni-coated Ti powder is significantly better than that of elemental powders. Because of this, next to the possibility for a decreased die wear, larger devices can be produced. Additional advantages are that no milling is required, thus avoiding contamination such as oxidation of Ti during the preparation stage, and that the intimate contact between Ni and Ti makes it possible to perform SHS with no or less preheating compared to green compacts made of elemental Ti and Ni powders.

The SHS process can produce Ni-Ti alloys with large pore volumes and a three-dimensional interconnected network of pores and channels. This porous network is particularly suitable for implants to achieve secure tissue-to-implant bonding. Pre-alloyed powder, such as atomised Ni-Ti, does not work for the SHS process, since it is already an alloy and the exothermic reaction does not take place during sintering.

During the manufacturing the coated powder, 50 to 500 g/l ammonium salts, such as (NH₄)₂SO₄ or (NH₄)₂CO₃, may be added, to prevent the formation of unwanted Ni(OH)₂ and to ensures a smooth coating.

The following Figures illustrate the invention.
Figure 1 shows an SEM of coated product.
Figure 2 shows an EDS map of the cross section of Ni-coated Ti powder; the solid Ti cores (left) and the Ni-coating (right) are visible.
Figure 3 gives a longitudinal view of powder A after compaction and SHS; the arrow indicates the direction of the propagation front.
Figure 4 is a SEM-image of powder A (left) and powder B (right) after SHS.
Figure 5 shows an XRD spectrum of Ni-coated powder D after SHS.
Figure 6 shows macroscopic pictures of samples made by the SHS process using different raw materials: (a) using powder D, (b) powder E, (c) powder F, (d) powder G.
Figure 7 shows SEM pictures of samples made by SHS using various raw materials at low (top) and high (bottom) magnification: (a) and (d) for powder D, (b) and (e) for powder F, (c) and (f) for powder G.

### Preferred embodiment 1

Ti powder along with a Ni bearing solution, such as a sulphate or a carbonate, and, in particular when a sulphate is used, ammonium hydroxide (ammoniac), preferably in a NH₃:Ni ratio of 2:1, are fed to an autoclave. A surface-active additive, such as anthraquinone, is also added to the solution to an amount of 0.2 to 5 wt.% of the Ti powder. This ensures a smooth coating of the Ti particles. The Ni is then precipitated on the titanium surface using H₂ at a temperature of 100 to 200 °C and at an H₂ pressure of 1.4 to 3.4 MPa. After coating, the slurry is washed, filtered and dried.

### Example 1

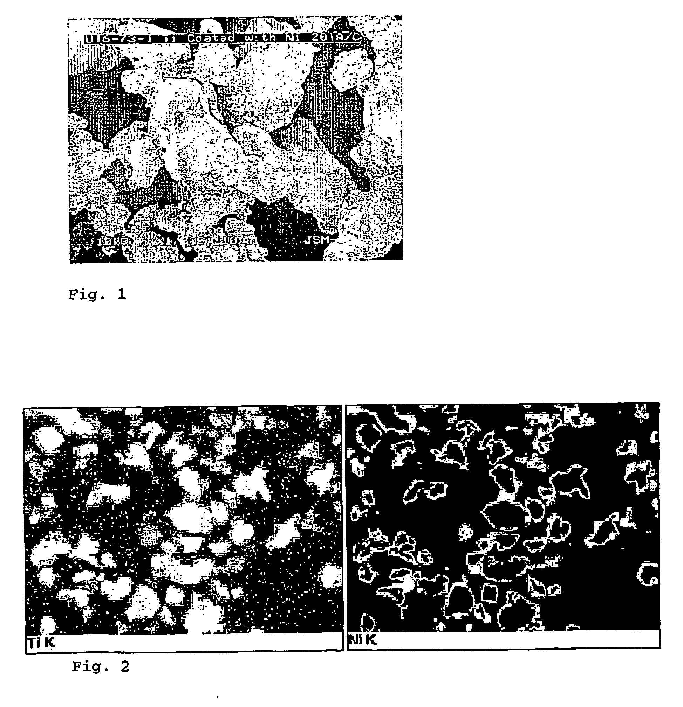

The result of coating a batch of Ti powder as described in preferred embodiment 1 is given in Table 1. The reduction temperature was 150 °C and the reduction pressure was maintained at 3.4 MPa. A SEM (Scanning Electron Microscope) picture of the coated product is shown in Figure 1. An EDS (Energy Dispersive Spectroscopy) map of the cross section of the powder is shown in Figure 2. SEM and EDS maps show a homogeneous and smooth coating.

**Table 1: Results of coating**

| Feed | | Coated powder | |
|---|---|---|---|
| Ni (g/L) | Ti (g/L) | Ni wt.% | Ti wt.% |
| 21.8 | 21.6 | 53.2 | 46.6 |

### Example 2

Ni-coated Ti powder was produced starting from 3 types of Ti powder having a different particle size distribution:
- powder A: Ni coated -400 mesh Ti powder;
- powder B: Ni coated -250 +325 mesh Ti powder;
- powder C: Ni coated -150 +200 mesh Ti powder.

The composition of the coated powder is shown in Table 2.

**Table 2: Composition of the coated powder**

| Powder reference | Composition | |
|---|---|---|
| | Ni wt.% | Ti wt.% |
| A | 53.8 | 45.8 |
| B | 53.7 | 46.1 |
| C | 53.3 | 46.0 |

### Example 3

The three different powders were die-compacted on an Instron-press to a density of respectively 48 %, 59 % and 51 % of the theoretical density using a compaction load of 22 kN, 19 kN and 11 kN respectively.

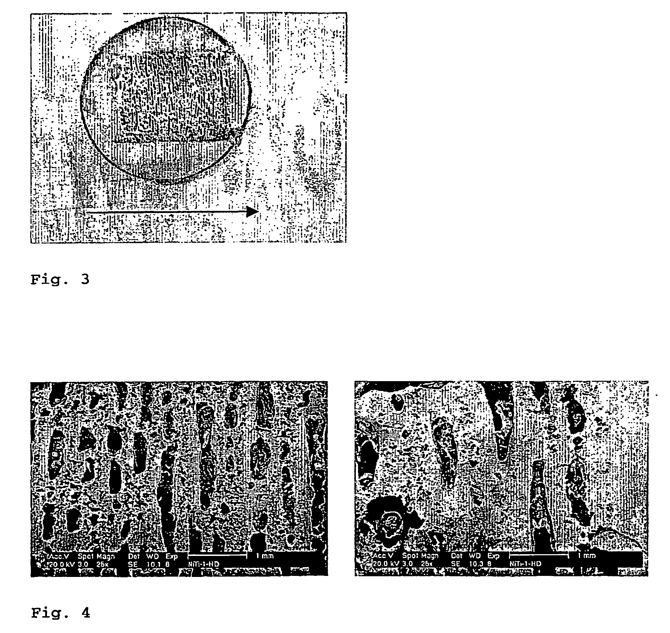

SHS performed on compacted powder A requires an ignition time of less than 10 seconds. The propagation front is parallel and stable and the resulting sample dimensions are also stable (Figure 3). Powders B and C showed a tendency to more intensive melting in the upper part of the sample.

Two types of pores are present: small ones and large elongated ones perpendicular to the propagation front direction (Figure 4). As the initial particle size increases from powder A to powder C, the width of the elongated pores increases from roughly 200-300 µm to 400-600 µm and finally to 800-1000 µm. The porosity distribution in each sample is homogeneous, except in the regions where a large amount of liquid phase was present, resulting in lower porosity.

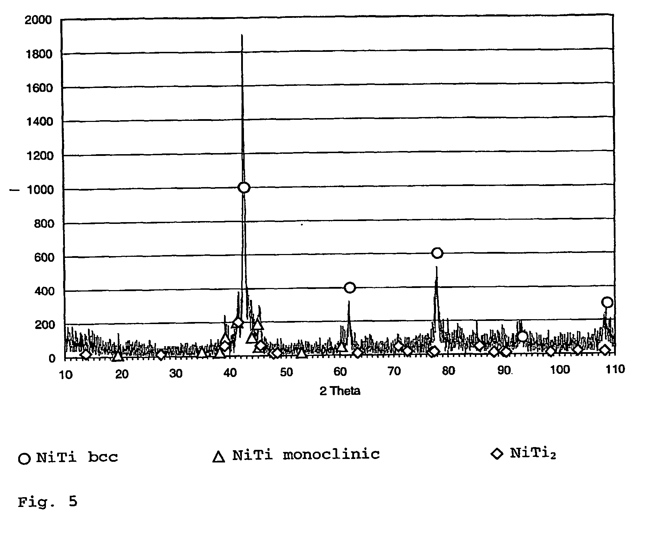

The phases present in the SHS-product have been determined using XRD (X-Ray Diffraction) and EDX (Energy Dispersive X-ray) analysis. The XRD diagram in Figure 5 clearly shows the presence of the desired Ni-Ti phase, both monoclinic and cubic, and possibly a limited amount of NiTi₂.

### Example 4

To be able to compare Ni-coated Ti powder with elemental Ni and Ti powders, the following batches were prepared:
- powder D: Ni coated -400 mesh Ti powder, blended with some additional Ni powder;
- powder E: Ni coated -250 +325 mesh Ti powder, blended with some additional Ni powder;
- powder F: Ni coated -150 +200 mesh Ti powder, blended with some additional Ni powder;
- powder G: Ni powder of 1.2 µm (d₅₀), mixed with -250 +325 mesh Ti powder in a 1:1 atomic ratio (55.07:44.93 Ni:Ti wt.% ratio).

Based on the composition analysis of the Ni-coated Ti powder, additional fine Ni powder was blended with the coated powder to balance the Ni:Ti atomic ratio to 1:1. The addition of Ni powder is shown in Table 3.

**Table 3: Amount of Ni added to 100 g of Ni-coated Ti powder**

| Powder reference | Ni:Ti (wt.%) | Ni powder added (g) |
|---|---|---|
| D | 53.8:45.8 | 2.34 |
| E | 53.7:46.1 | 2.80 |
| F | 53.3:46.0 | 3.08 |

Quartz tubes with a diameter of 20 to 25 mm and a length of 130 to 170 mm were used for containing the powder. Powder mixture G was ball milled for 2 hours before being loosely packed in a quartz tube. Green density of the mixed powder was about 50 to 60 %. A load of 30 to 40 kN was needed to press the sample.
The green densities for powders D, E and F were respectively about 45%, 50% and 65%, accomplished using loads of 10 kN, 15 kN and 18 kN respectively.

All samples were placed in a vacuum chamber with a vacuum of about 0.01 Pa. After pre-heating the samples to 350 °C for 1 hour, the samples were ignited. SHS took place.

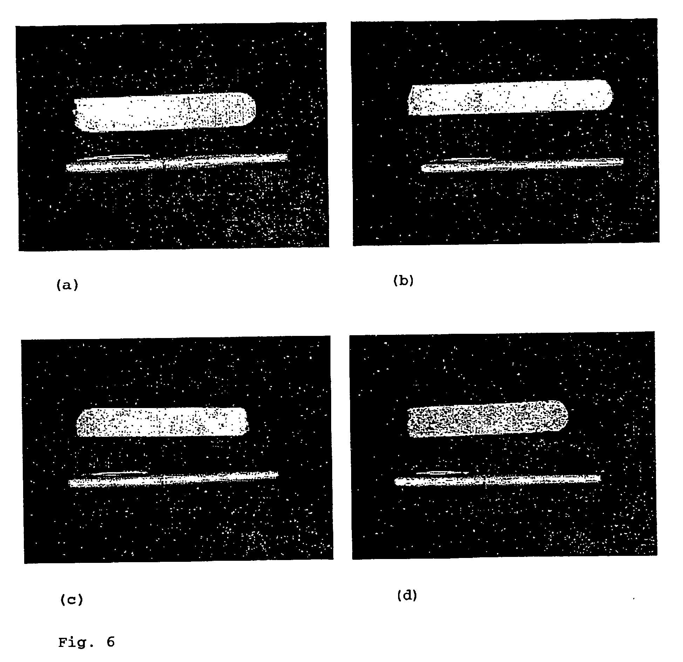

Figure 6 shows macroscopic pictures of the samples prepared by SHS. The surface morphology of the samples made by Ni-coated Ti powder was homogeneous. The surface morphology of the samples made by mixed Ni and Ti powders was rough and the porosity was inhomogeneous.

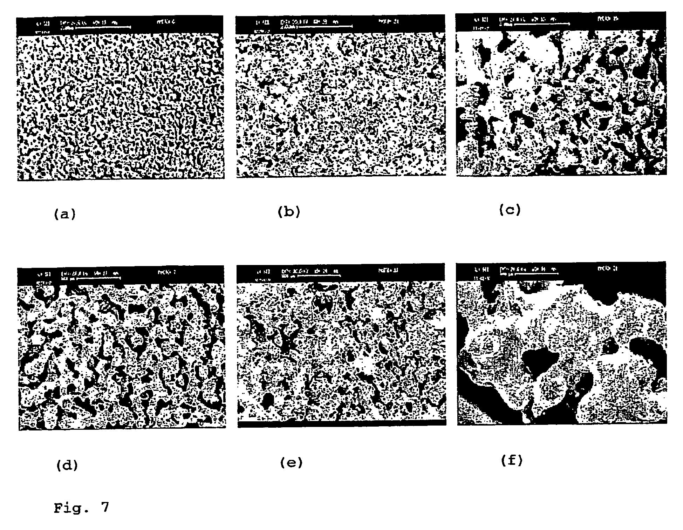

SEM pictures in Figures 7(c) and 7(f) show that the pore size and morphology of the sample made from mixed Ni and Ti powder are inhomogeneous. Figures 7(a), 7(b), 7(d), and 7(e) show that the pore size and morphology prepared from finer Ni-coated Ti powders are more homogeneous than those by coarser Ni-coated Ti powder. There are also more open pores in the samples using finer Ni-coated Ti powders. Overall, samples using Ni-coated Ti powder have a more homogeneous porosity than that using mixed Ni and Ti powders.

## Claims

1. Coated powder, having a metallic Ti core and a metallic Ni coating, said coated powder is **characterised by** a Ni:Ti atomic ratio of more than 0.5, preferably between 0.9 and 1.1, and more preferably between 0.96 and 1.04.

2. Powder mixture comprising a coated powder according to claim 1, and further comprising one or both of Ni-bearing powder and Ti-bearing powder, wherein the Ni:Ti atomic ratio of the mixture is between 0.9 and 1.1, preferably between 0.99 and 1.01.

3. Coated powder or powder mixture according to any one of claims 1 and 2, **characterised by** a particle size finer than 150 mesh.

4. Use of a coated powder or a powder mixture according to any one of claims 1 to 3 for the manufacture of a sintered body.

5. Use of a coated powder or a powder mixture according to claim 4. **characterised in that** the sintered body is obtained by a self-propagating high temperature process.

6. A sintered body obtained by a self-propagating high temperature process using powders according to any one of claims 1 to 3.

7. process of manufacturing a coated powder according to claim 1, comprising the steps of:
- providing for suitable quantities of a Ti powder and of a Ni salt bearing aqueous solution;
- feeding said powder and said solution in an autoclave together, with a quantity of NH₄OH, and, optionally, with a quantity of ammonium salts;
- precipitating the Ni onto the Ti powder by hydrogen reduction;
- washing, filtering and drying the slurry obtained, thereby obtaining a Ki-coated Ti powder.

8. Process according to claim 7, whereby the Ni is precipitated onto the Ti powder at a temperature of at least 100 °C and a hydrogen pressure in the autoclave of at least 1.4 MPa.

9. Process of manufacturing a coated powder according to claim 2, comprising the steps of claim 7, and further comprising the step of intimately mixing the Ni-coated Ti powder with one or both of Ni-bearing and Ti-bearing powder.

10. Process of manufacturing a porous sintered body based on a Ni-Ti alloy, comprising the steps of any one of claims 7 to 9, and further comprising the step of subjecting the powder or powder mixture to a self-propagating high temperature synthesis operation.

11. A sintered body obtained by a process according to claim 10.

## Patentansprüche

1. Beschichtetes Pulver mit einem metallisches Ti enthaltenden Kern und einer metallisches Ni enthaltenden Beschichtung, **gekennzeichnet durch** ein Ni:Ti-Atomverhältnis von mehr als 0,5, vorzugsweise zwischen 0,9 und 1,1 und besonders bevorzugt zwischen 0,96 und 1,04.

2. Pulvermischung, enthaltend ein beschichtetes Pulver nach Anspruch 1 und außerdem Ni enthaltendes Pulver und/oder Ti enthaltendes Pulver, wobei das Ni:Ti-Atomverhältnis der Mischung zwischen 0,9 und 1,1 und vorzugsweise zwischen 0,99 und 1,01 liegt.

3. Beschichtetes Pulver oder Pulvermischung nach Anspruch 1 oder 2, **gekennzeichnet durch** eine Teilchengröße von weniger als 150 Mesh.

4. Verwendung eines beschichteten Pulvers oder einer Pulvermischung nach einem der Ansprüche 1 bis 3 zur Herstellung eines Sinterkörpers.

5. Verwendung eines beschichteten Pulvers oder einer Pulvermischung nach Anspruch 4, **dadurch gekennzeichnet, daß** der Sinterkörper nach einem SHS-Verfahren (SHS = self-propagating high temperature synthesis) erhalten wird.

6. Sinterkörper, erhalten nach einem SHS-Verfahren mit Pulvern nach einem der Ansprüche 1 bis 3.

7. Verfahren zur Herstellung eines beschichteten Pulvers nach Anspruch 1, bei dem man:
- geeignete Mengen eines Ti-Pulvers und einer ein Ni-Salz enthaltenden wäßrigen Lösung bereitstellt;
- das Pulver und die Lösung zusammen mit einer Menge von NH₄OH und gegebenenfalls mit einer Menge von Ammoniumsalzen einem Autoklaven zuführt;
- das Ni durch Wasserstoffreduktion auf das Ti-Pulver auffällt;
- die erhaltene Aufschlämmung wäscht, filtriert und trocknet, wobei man ein Ni-beschichtetes Ti-Pulver enthält.

8. Verfahren nach Anspruch 7, bei dem man das Ni bei einer Temperatur von mindestens 100°C und einem Wasserstoffdruck im Autoklaven von mindestens 1,4 MPa auf das Ti-Pulver auffällt.

9. Verfahren zur Herstellung eines beschichteten Pulvers nach Anspruch 2 mit den Schritten gemäß Anspruch 7, bei dem man ferner das Ni-beschichtete Ti-Pulver innig mit Ni enthaltendem Pulver und/oder Ti enthaltendem Pulver vermischt.

10. Verfahren zur Herstellung eines porösen Sinterkörpers auf Basis einer Ni-Ti-Legierung mit den Schritten gemäß einem der Ansprüche 7 bis 9, bei dem man ferner das Pulver oder die Pulvermischung einem SHS-Arbeitsgang unterwirft.

11. Sinterkörper, erhalten durch ein Verfahren nach Anspruch 10.

## Revendications

1. Poudre enrobée qui présente un coeur métallique en Ti et un enrobage métallique en Ni, ladite poudre enrobée étant **caractérisée par** un rapport atomique Ni : Ti supérieur à 0,5, de préférence compris entre 0,9 et 1,1 et de manière plus préférable entre 0,96 et 1,04.

2. Mélange de poudres qui comprend une poudre enrobée selon la revendication 1 et qui comprend en outre une poudre qui contient du Ni, une poudre qui contient du Ti ou les deux, le rapport atomique Ni : Ti du mélange étant compris entre 0,9 et 1,1 et de préférence entre 0,99 et 1,01.

3. Poudre enrobée ou mélange de poudres selon l'une quelconque des revendications 1 et 2, **caractérisés par** une taille de particule plus fine que celle qui correspond à une maille de 150.

4. Utilisation d'une poudre enrobée ou d'un mélange de poudres enrobées selon l'une quelconque des revendications 1 à 3 dans la fabrication d'un corps fritté.

5. Utilisation d'une poudre enrobée ou d'un mélange de poudres selon la revendication 4, **caractérisée en ce que** le corps fritté est obtenu à l'aide d'une opération de synthèse par auto-propagation à haute température.

6. Corps fritté obtenu à l'aide d'une opération de synthèse par auto-propagation à haute température qui utilise des poudres selon l'une quelconque des revendications 1 à 3.

7. Procédé pour préparer une poudre enrobée selon la revendication 1, le procédé comprenant les étapes qui consistent à :
prévoir en quantités appropriées une poudre de Ti et une solution aqueuse qui contient un sel de Ni,
amener ladite poudre et ladite solution dans un autoclave avec une quantité de NH₄OH et facultativement avec une quantité de sels d'ammonium,
précipiter le Ni sur la poudre de Ti par réduction à l'hydrogène,
rincer, filtrer et sécher la suspension obtenue, ce qui permet d'obtenir une poudre de Ti enrobée de Ni.

8. Procédé selon la revendication 7, dans lequel le Ni est précipité sur la poudre de Ti à une température d'au moins 100°C et sous une pression d'hydrogène dans l'autoclave d'au moins 1,4 MPa.

9. Procédé pour préparer une poudre enrobée selon la revendication 2, le procédé comprenant les étapes selon la revendication 7 et en outre l'étape qui consiste à mélanger intimement la poudre de Ti enrobée de Ni avec une poudre qui contient du Ni, une poudre qui contient du Ti ou les deux.

10. Procédé pour fabriquer un corps fritté poreux à partir d'un alliage Ni-Ti, le procédé comprenant les étapes selon l'une quelconque des revendications 7 à 9 et en outre l'étape qui consiste à soumettre la poudre ou le mélange de poudres enrobées à une opération de synthèse par auto-propagation à haute température.

11. Corps fritté obtenu à l'aide du procédé selon la revendication 10.
